# FASCICULE DE BREVET EUROPEEN

(11) **EP 1 565 114 B1**
(45) Date de publication et mention de la délivrance du brevet: **09.01.2008**
(21) Numéro de dépôt: 03812206.5
(22) Date de dépôt: 18.11.2003
(51) Int. Cl.: A61B 17/34, A61M 25/06

(54) **DISPOSITIF DE CHIRURGIE ENDOVASCULAIRE**
ENDOVASKULÄRE CHIRURGISCHE VORRICHTUNG
ENDOVASCULAR SURGERY DEVICE

(30) Priorité: 28.11.2002 FR 0214931
(43) Date de publication de la demande: 24.08.2005
(73) Titulaire: Formichi, Maxime, 13007 Marseille (FR)
(72) Inventeur: Formichi, Maxime, 13007 Marseille (FR)
(74) Mandataire: Marek, Pierre
(86) Numéro de dépôt international: PCT/FR2003/003419
(87) Numéro de publication internationale: WO 2004/049960

(56) Documents cités:
- EP-A- 0 583 049
- EP-A- 0 608 985
- US-A- 4 385 637
- US-A- 5 628 734
- US-A- 5 858 002

## Description

La présente invention concerne un dispositif chirurgical utile pour la chirurgie endovasculaire, y compris la radiologie interventionnelle.

Plus particulièrement, la présente invention concerne un dispositif pouvant être mis en oeuvre par voie mini-invasive, notamment par voie laparoscopique et/ou coelioscopique, notamment au niveau des gros vaisseaux intra-abdominaux.

Actuellement, la chirurgie endovasculaire se pratique dans les buts et conditions suivantes.

On cherche à introduire des cathéters, notamment à ballonnet et des endoprothèses en vue du traitement des lésions sténosantes ou d'anévrismes artériels.

Ces interventions se pratiquent soit par voie percutanée, avec différents sites de ponction possibles, soit par abord chirurgical d'une artère, à savoir le plus souvent l'artère fémorale.

L'introduction des endoprothèses est réalisée à l'aide de cathéters introduits à l'intérieur des vaisseaux. L'introduction par voie percutanée ne peut se réaliser que pour des tailles de cathéters réduites, notamment de diamètre inférieur à environ 3 à 3,60mm (10 à 12 French)..

Lorsque l'endoprothèse requiert l'usage de cathéter de plus grand diamètre, l'abord au niveau d'une artère est obligatoire, et dans la très grande majorité des cas, on utilise l'artère fémorale..

En pratique, on réalise une ponction de l'artère à l'aide d'une aiguille creuse, et il se produit alors une hémorragie sous forme d'un jet de sang qui indique que l'aiguille est bien dans la lumière artérielle et peut être contrôlé dans la mesure où l'on intervient par voie percutanée, et donc sous contrôle de la vue. Puis l'on introduit un guide, dit «guide de radiologie» constitué d'un fil flexible à extrémité souple, à l'intérieur de l'aiguille creuse, lequel permet ultérieurement d'introduire les cathéters nécessaires à l'injection de diverses substances utiles pour un usage thérapeutique ou diagnostic, mais surtout utile pour l'introduction de cathéter à ballonnet et/ou d'une endoprothèse.

Les interventions au niveau des artères fémorales présentent certains inconvénients. Tout d'abord, le délai de cicatrisation impose au moins 5 jours d'hospitalisation lorsque l'on a fait une ouverture chirurgicale. D'autre part, la taille des cathéters reste limitée à la taille de l'artère fémorale, soit environ de 7,2 à 8,4 mm (24 à 28 French). Alors que certains vaisseaux intra-abdominaux, de diamètre plus importants, permettraient l'introduction de cathéters de plus grand diamètre.

Enfin, chez certains patients qui ont des artères iliaques sténosées, tortueuses et/ou calcifiées, il peut être difficile, voire impossible de faire progresser le cathéter à l'intérieur jusqu'au site de délivrance de l'endoprothèse.

Actuellement, pour les interventions par voie percutanée ou par abord fémoral, on utilise des aiguilles creuses qui permettent de réaliser la ponction de l'artère et d'introduire manuellement le guide à l'intérieur de l'aiguille jusque dans la lumière artérielle sur une distance qui varie en fonction du site à atteindre, au minimum de 20 à 30 cm. Pendant le temps d'introduction de l'aiguille, il se produit une hémorragie. Ensuite, après mise en place du guide, on retire l'aiguille, le guide étant laissé en place. Puis, on enfile et fait progresser ledit cathéter pardessus ledit guide qui se trouve donc à l'intérieur du cathéter, et fait donc fonction de guide du cathéter jusqu'à son extrémité implantée à l'intérieur des vaisseaux.

Pour faciliter l'introduction ultérieure des cathéters et endoprothèses, on met tout d'abord en place un introducteur à valve qui consiste en un tuyau en plastique et relativement rigide, faisant office de gaine de protection, surmonté par une capsule d'étanchéité comprenant une membrane souple pouvant être perforée par lesdits cathéters et permettant d'introduire les cathéters de manière étanche. En général, ces capsules d'étanchéité comportent en outre une ouverture latérale se terminant par une vanne ou robinet permettant l'injection de différents produits dans le sang et/ou le rinçage régulier de l'intérieur de l'introducteur.

Les guides utilisés dans ces procédures endovasculaires sont appelés «guides de radiologie» indépendamment du fait qu'ils sont utilisés également pour des interventions à caractère plus chirurgical que radiologique, car à l'origine, ces guides étaient utilisés pour la mise en place de cathéters ou sondes de radiologie. qui servaient uniquement à injecter des substances médicamenteuses ou des produits dits de contraste utilisés pour obtenir une radiographie des artères ou artériographie.

Ces guides sont communément réalisés en matière synthétique flexible avec une âme élastique recouverte d'une surface souple et non thrombogène, évitant les plicatures, d'un diamètre de 0,35 à 0,97 mm (0,014 à 0,038 pouces).

Par ailleurs, on connaît la chirurgie par voie laparoscopique ou coelioscopique qui est réalisée à travers la paroi abdominale en introduisant des guides cylindriques creux appelés trocarts, de diamètre variant de 5 à 12 mm, ces trocarts permettant d'introduire ensuite des instruments opératoires et des moyens de visualisation tels qu'une caméra pour réaliser une opération de manière vidéo-assistée, à l'intérieur de l'abdomen notamment, ainsi que d'insuffler du gaz (en général CO₂) l'air dans l'abdomen pour agrandir l'espace de travail.

Mais, la réalisation de procédure endovasculaire après un abord laparoscopique, notamment au niveau des vaisseaux intra-abdominaux, n'est pas réalisable actuellement pour les raisons suivantes.

Lors d'une ponction avec une aiguille d'un gros vaisseau à l'intérieur de l'abdomen, il se produirait nécessairement une hémorragie qui ne pourrait pas être contrôlée comme sous contrôle de la vue. Cette hémorragie représente bien évidemment un danger pour le patient. En outre, il n'est pas possible d'aspirer le sang au risque d'aspirer en même temps le gaz introduit initialement pour réaliser l'opération par voie laparoscopique après gonflement de l'abdomen. Il en résulte que le sang absorbant la lumière, il devient difficile, voire impossible de visualiser les opérations et donc impossible en pratique de réaliser une procédure endovasculaire dans ces conditions.

Le but de la présente invention est donc de fournir un nouveau dispositif permettant de réaliser des procédures endovasculaires par voie laparoscopique.

Plus précisément, un but de la présente invention est de fournir un dispositif permettant de ponctionner une artère en contrôlant l'hémorragie et d'introduire à l'intérieur un guide par voie laparoscopique, puis de permettre ainsi l'introduction endovasculaire ultérieure de cathéters et d'endoprothèses de taille plus volumineuse par voie laparoscopique.

Pour ce faire, la présente invention fournit un dispositif chirurgical permettant la ponction d'un vaisseau, notamment d'une artère, et l'introduction endovasculaire d'un guide de radiologie, voire d'un cathéter dont le diamètre externe serait inférieur au diamètre interne de l'aiguille, destiné à être utilisé en chirurgie endovasculaire et laparoscopique ou coelioscopique, notamment au niveau des vaisseaux intra-abdominaux, caractérisé en ce qu'il comprend un premier tuyau transparent souple coopérant à son extrémité distale avec une aiguille métallique creuse à laquelle il est raccordé, et dans laquelle on peut faire passer un dit guide de radiologie, ledit premier tuyau comprenant à son extrémité proximale un moyen d'obturation dudit premier tuyau, ainsi qu'un moyen d'introduction permettant l'introduction étanche dans ledit premier tuyau d'un dit guide de radiologie.

On comprend que ledit premier tuyau transparent souple est de dimensions suffisantes pour :
■ assurer la jonction entre ledit vaisseau et la surface cutanée avec une partie dudit premier tuyau transparent dépassant à l'extérieur du patient, lorsque ladite ponction vasculaire est réalisée, et
■ contenir le jet de sang s'échappant dudit vaisseau lors de ladite ponction vasculaire.

Le dispositif selon la présente invention permet la ponction d'un vaisseau et notamment d'une artère de gros calibre après abord coelioscopique en évitant toute hémorragie interne, comme c'est le cas avec une aiguille classique. Il est donc particulièrement utile pour réaliser une ponction sous coelioscopie car la moindre .hémorragie peut dans ce cas entraîner une conversion chirurgicale.

En effet, ledit premier tuyau transparent souple fait fonction de réservoir du sang s'écoulant du vaisseau après ponction par ladite aiguille sans entraîner d'hémorragie dans le champ de vision de l'optique utilisée pour visualiser l'intervention laparoscopique. En outre, de par le fait que ledit tuyau est transparent, il permet de visualiser la couleur du liquide contenu à l'intérieur et donc de reconnaître un reflux sanguin et contrôler ainsi la réussite de la ponction.

Ledit premier tuyau transparent permet également d'acheminer le guide de radiologie que l'on doit introduire à l'aide du dispositif selon l'invention pour l'acheminer depuis la surface cutanée jusqu'à ladite aiguille lorsque celle-ci est en place à l'intérieur dudit vaisseau. La transparence dudit premier tuyau permet également de contrôler que le passage des guides de radiologie et des sondes lors de leur progression en direction de l'aiguille se fait correctement.

On comprend que la longueur du dit premier tuyau transparent dépend de l'emplacement du vaisseau à ponctionner et des caractéristiques anatomiques du patient.

Enfin, la souplesse dudit premier tuyau transparent permet de le courber en fonction de l'orientation du vaisseau à ponctionner.

Le dispositif selon l'invention permet également, après ponction toutes les manoeuvres des guides de radiologie, telles que leur introduction et retrait depuis l'extérieur du patient vers la lumière artérielle, en dehors du champ de la coelioscopie, en extra pariétal comme dans une procédure endovasculaire classique.

Ledit premier tuyau souple permet également d'injecter des produits à visée diagnostique ou thérapeutique dans les vaisseaux abordés par coelioscopie par des manoeuvres extra pariétales, hors du champ de coelioscopie.

Une fois que le dispositif a permis les manoeuvres nécessaires à la mise en route de la procédure endovasculaire, il peut être retiré sans avoir à enlever les guides et sondes de radiologie introduits dans ledit vaisseau par son intermédiaire.

Le dispositif selon l'invention permet donc le développement de nouvelles techniques chirurgicales associant coelioscopie ou laparoscopie et gestes endovasculaires, mais il peut aussi être utilisé pour une ponction par voie percutanée ou sous contrôle de la vue après abord chirurgical.

Selon une autre caractéristique originale et avantageuse de la présente invention, ladite aiguille présente un profil longitudinal courbe.

Plus particulièrement, la courbure de ladite aiguille correspond à une inclinaison de son extrémité distale par rapport à son extrémité proximale solidaire dudit élément de raccordement, d'un angle de 10 à 45°.

On entend ici par «inclinaison» l'angle d'inclinaison entre les tangentes aux 2 extrémités proximale et distale de ladite aiguille.

Cette courbure de l'aiguille est particulièrement avantageuse dans une intervention endovasculaire laparoscopique, car elle permet de faciliter l'introduction de l'aiguille dans le vaisseau de telle sorte que le biseau pointu de l'aiguille soit dirigé dans l'axe du vaisseau sans perforer la paroi opposée du vaisseau. En effet, alors qu'en chirurgie endovasculaire abordée par voie percutanée l'aiguille est maintenue par les tissus sous cutanés entourant les vaisseaux il n'est pas nécessaire de rentrer entièrement celle-ci dans le vaisseau, en chirurgie par voie laparoscopique; notamment en intra abdominale, les vaisseaux sont dénudés et l'aiguille n'étant pas retenue par des tissus avoisinants, il est nécessaire d'introduire l'aiguille plus complètement dans le vaisseau sans piquer la paroi opposée.
Dans un mode de réalisation particulier, ladite aiguille creuse comprend :
■ une partie distale à extrémité pointue et comprenant un premier canal interne longitudinal creux, de préférence à section transversale circulaire, à travers lequel on peut faire passer un dit guide de radiologie, et
■ une partie proximale formant un élément de raccordement de ladite aiguille et dudit premier tuyau transparent, ledit élément de raccordement comportant un deuxième canal interne creux assurant la communication entre l'intérieur dudit premier tuyau transparent et ledit premier canal creux de ladite aiguille et le diamètre interne dudit deuxième canal interne de l'élément de raccordement étant au moins égal au diamètre dudit premier canal creux interne.

Dans un mode de réalisation avantageux, ledit premier tuyau transparent présente un diamètre interne supérieur ou égal au diamètre interne dudit deuxième canal interne creux dudit élément de raccordement, lequel deuxième canal creux comprend une zone de transition à section transversale circulaire, de préférence en forme d'entonnoir, de diamètre diminuant progressivement jusqu'à rejoindre ledit premier canal interne creux de ladite aiguille.

La zone de transition à l'intérieur de l'élément de raccordement avec un changement de section progressif permet de diriger harmonieusement et sans heurt le guide de radiologie et les sondes de radiologie vers la lumière de l'aiguille.

La mise en oeuvre d'un premier tuyau transparent à diamètre relativement grand par rapport à celui dudit premier canal interne creux de l'aiguille permet de mettre en oeuvre des tuyaux avec une paroi suffisamment épaisse en matière synthétique, de sorte que ledit tuyau reste à la fois suffisamment solide et souple pour tolérer différentes courbures en évitant des plicatures éventuelles. D'autre part, il permet de contenir un volume sanguin suffisant correspondant au jet de sang initial sous forte pression s'échappant dudit vaisseau.

Bien entendu, la taille du diamètre externe dudit premier tuyau doit être inférieur au plus petit diamètre des trocarts mis en oeuvre lors de l'intervention. En pratique, les trocarts chirurgicaux disponibles dans le commerce ont un diamètre interne en général supérieur à 5 mm, de sorte qu'un dit premier tuyau transparent de diamètre externe inférieur à 4 mm pourra convenir.

Par ailleurs, le diamètre interne dudit premier tuyau doit être supérieur au diamètre des guides de radiologie, de préférence supérieur au plus gros guide de radiologie disponible dans le commerce, soit supérieur à environ 1 mm.

Dans un mode de réalisation particulier, ledit élément de raccordement comprend à son extrémité proximale un premier manchon tubulaire sur la surface extérieure duquel vient s'ajuster l'extrémité distale dudit premier tuyau souple transparent, ledit premier manchon tubulaire se prolongeant à son extrémité distale d'une partie intermédiaire assurant la jonction entre ledit premier manchon tubulaire et ladite partie distale pointue de l'aiguille, de telle sorte que ladite partie intermédiaire présente une section transversale circulaire de diamètre externe supérieur ou égal à celui du diamètre externe dudit premier manchon tubulaire, et diminuant progressivement depuis sa section transversale de plus grand diamètre jusqu'à sa section transversale au niveau de la jonction avec ladite partie distale à extrémité pointue de l'aiguille, ledit deuxième canal interne creux dudit élément de raccordement comprenant à l'intérieur de ladite partie intermédiaire ladite zone de transition à section transversale diminuant progressivement en forme d'entonnoir.

Le profil externe de ladite partie intermédiaire de l'élément de raccordement dont la section transversale diminue progressivement, permet de faciliter l'acheminement de ladite aiguille et dudit dispositif dans son entier à travers le trocart de laparoscopie ou coelioscopie en évitant les coincements au niveau de l'aiguille et/ou de l'élément de raccordement ainsi que les pliures ou torsions indésirables au niveau dudit premier tuyau transparent lors du passage dans ledit trocart lorsque ledit dispositif est introduit par un trocart de laparoscopie.

Selon une autre caractéristique avantageuse de la présente invention, ledit premier tuyau transparent comprend à son extrémité proximale une capsule étanche assurant l'obturation dudit premier tuyau souple, ladite capsule comportant une membrane souple en matériau élastique incisée, apte à être traversée de manière étanche par un dit guide de radiologie.

Cette capsule étanche fait office de vanne anti-reflux sanguin en permettant l'introduction des guides de radiologie sans fuite sanguine extériorisée au niveau de la capsule. On peut utiliser notamment comme dite membrane souple une membrane en silicone.

De telles capsules étanches sont connues de l'homme de l'art et disponibles dans le commerce pour équiper les introducteurs semi-rigides utilisés en chirurgie percutanée endovasculaire.

On entend ici par «membrane incisée apte à être traversée de manière étanche» qu'il n'y a pas de fuite vers l'extérieur du liquide, notamment du sang contenu dans ledit premier tuyau, lors de l'introduction dudit guide de radiologie à travers l'incision de la membrane et après ladite introduction du guide. On comprend que la surface externe du guide est coincée d'une manière étanche par le matériau élastique constitutif de la membrane, au niveau de l'incision.

Dans un mode de réalisation particulier, la membrane est pré-incisée avec une incision en forme de croix.

Dans un mode de réalisation particulier, ledit premier tuyau souple comporte un moyen d'injection permettant d'injecter un liquide dans ledit premier tuyau souple, de préférence constitué d'un orifice latéral dans la zone proximale dudit premier tuyau destinée à rester à l'extérieur du patient, ledit orifice latéral étant de préférence intégré, le cas échéant, à une dite capsule étanche.

Avantageusement, ledit moyen d'injection comprend un second tuyau souple adaptable sur ledit orifice latéral et comportant à son extrémité libre un robinet de préférence à plusieurs voies.

Le dispositif selon l'invention peut se présenter sous la forme d'un ensemble ou kit comprenant différents éléments tels que :
- ledit premier tuyau transparent souple,
- ladite aiguille métallique creuse,
- le cas échéant, ledit moyen d'obturation, de préférence ladite capsule étanche,
- le cas échéant, ledit moyen d'injection, de préférence ledit second tuyau souple, comprenant, de préférence encore, ledit robinet.

Ces différents éléments peuvent être préassemblés ou désassemblés , au moins pour partie, dans des conditionnements séparés, ou de préférence dans un même conditionnement, notamment en vue d'un assemblage ultérieur avant utilisation.

D'autres caractéristiques et avantages de la présente invention apparaîtront à la lumière de la description détaillée qui va suivre, faite en référence aux figures 1 à 4 dans lesquelles :
- la figure 1 représente une vue schématique d'un dispositif selon l'invention,
- la figure 2 représente une vue d'un dispositif selon l'invention comportant une capsule étanche 4 munie d'un orifice latéral 5,
- la figure 3 représente une vue d'une coupe longitudinale de ladite partie métallique terminale comprenant ladite aiguille d'un dispositif selon l'invention, et
- la figure 4 représente une vue d'une coupe longitudinale d'une capsule étanche 4.

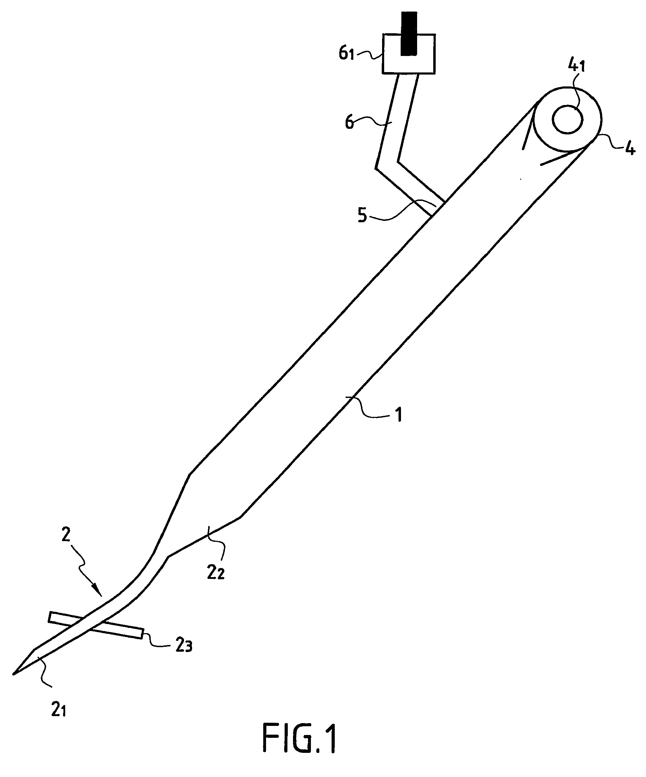

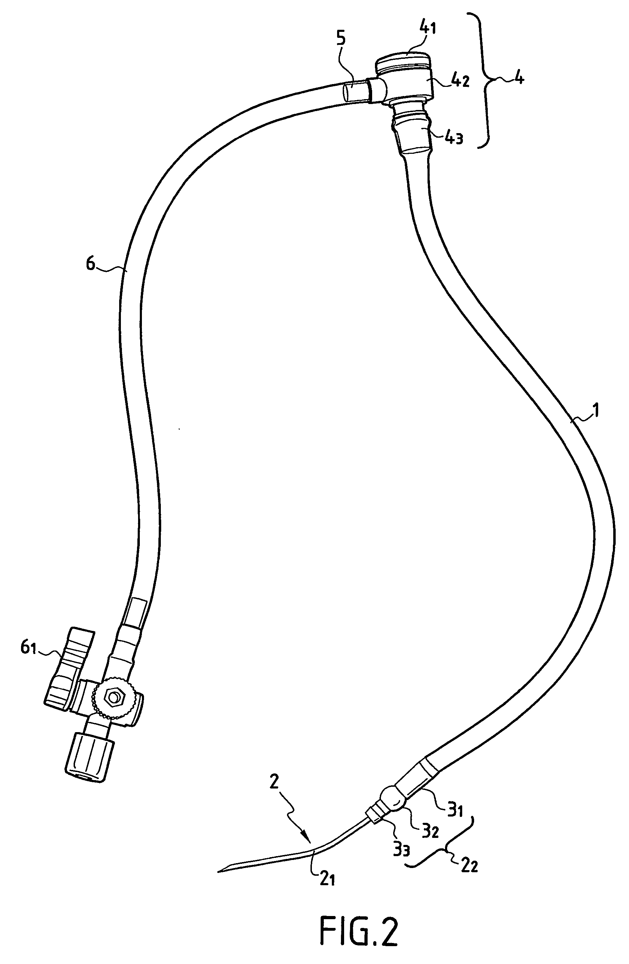

Le dispositif selon l'invention, tel que représenté sur les figures 1 et 2, comprend :
- un dit premier tuyau souple transparent 1, qui, à titre illustratif, présente une longueur de 20 à 50 cm et un diamètre externe de 2 à 5 mm, et
- une aiguille creuse 2 comprenant une partie distale métallique creuse courbe 2₁ comprenant un premier canal interne, et une partie proximale faisant fonction d'élément de raccordement 2₂ avec ledit premier tuyau transparent 1, ledit élément de raccordement comprenant un deuxième canal interne creux.

L'extrémité distale dudit premier tuyau 1 en PVC vient s'ajuster en force sur la surface externe d'un manchon tubulaire 3₁ constituant la partie proximale dudit élément de raccordement 2₂. lors de son assemblage.

L'élément de raccordement 2₂ comprend une partie intermédiaire 3₂ creuse constituée par un renflement présentant un profil externe en forme arrondie, de diamètre externe supérieur au diamètre externe dudit premier manchon tubulaire 3₁. Ledit renflement 3₂ se situe dans le prolongement dudit premier manchon tubulaire 3₁ avec lequel il vient de matière.

Ledit élément de raccordement 2₂ comporte un deuxième manchon tubulaire 3₃ situé par rapport audit renflement 3₂ à l'opposé dudit premier manchon tubulaire 3₁ et il vient également de matière avec celui-ci.

Ladite aiguille est réalisée en acier inoxydable biocompatible.

Ledit deuxième manchon tubulaire 3₃ assure d'une part la jonction entre la partie distale pointue 2₁ de l'aiguille creuse 2₁ et l'élément de raccordement 2₂, et d'autre part, il permet d'y ajuster un petit tuyau en plastique souple (non représenté) venant recouvrir la partie distale pointue 2₁ de l'aiguille creuse 2₁, de façon à protéger celle-ci avant son utilisation pour éviter des piqûres pouvant détériorer son conditionnement ou blesser le personnel amené à la manipuler avant l'intervention.

Les dimensions de l'aiguille 2 sont adaptées en fonction de la taille du vaisseau à ponctionner et de la taille des guides de radiologie que l'on souhaite introduire ultérieurement.

La taille des guides de radiologie dépend principalement de l'emplacement des vaisseaux dans le corps et des cathéters que l'on souhaite introduire ultérieurement à l'aide de ce guide, lesquels dépendent également de la taille de l'endoprothèse ou autre objet que l'on souhaite introduire de manière endovasculaire ultérieurement.
A titre illustratif, on pourra utiliser en pratique des aiguilles 2 :
- de 2.à 5 cm de longueur,
- de 0,5 mm à 3 mm de diamètre externe,
- avec une courbure correspondant à un angle d'inclinaison de 20 à 30°, et
- de 0,35 mm à 2 mm de diamètre de dit premier canal interne creux.

Les dimensions dudit élément de raccordement 2₂ sont adaptées en fonction du diamètre interne dudit premier tuyau 1, lequel dépend de la longueur et donc de la morphologie du patient à opérer.

Ledit premier manchon tubulaire 3₁ présentera un diamètre externe sensiblement identique au diamètre interne dudit premier tuyau transparent 1. La longueur dudit premier manchon tubulaire 3₁ est en pratique et à titre illustratif de 5 à 10 mm pour un dit premier tuyau transparent de 3 à 5 mm de diamètre externe.

Ledit élément de raccordement 2₂ comporte un deuxième canal interne creux qui débute par l'intérieur dudit premier manchon tubulaire 3₁, traverse l'intérieur dudit renflement 3₂ et se termine par l'intérieur dudit deuxième manchon tubulaire 3₃.

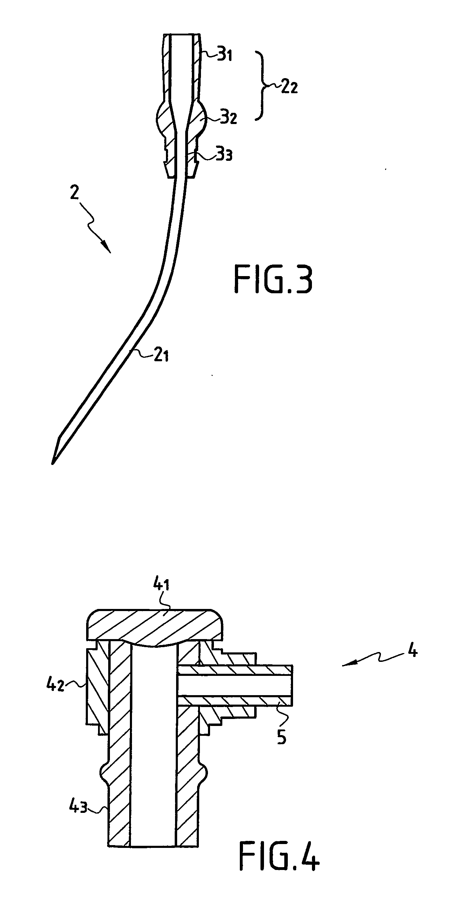

La section en coupe longitudinale de ladite aiguille 2 représentée sur la figure 3 montre que ledit deuxième canal interne forme un entonnoir avec une diminution progressive de son diamètre depuis l'extrémité distale dudit premier manchon tubulaire 3₁ jusqu'à l'extrémité distale dudit deuxième manchon tubulaire 3₃, lequel se prolonge par l'extrémité proximale dudit premier canal interne de la partie distale pointue 2₁ de l'aiguille.

L'extrémité proximale dudit premier tuyau 1 est assemblée sur une capsule étanche 4. Cette capsule étanche 4 comporte un compartiment central sensiblement cylindrique 4₂ dont l'orifice supérieur est recouvert par une membrane souple élastique 4₁ comportant une incision étanche en forme de croix, c'est-à-dire que le matériau constitutif de ladite membrane est suffisamment souple et résistante pour qu'il n'y ait pas de fuites rétrogrades de liquide sanguin au niveau de l'incision d'une part et que, d'autre part, l'incision permette d'introduire des guides de radiologie de 0,35 à 2 mm de diamètre sans fuite du liquide contenu dans ledit premier tuyau à la jonction entre ledit guide de radiologie et la membrane 4₁.Ledit compartiment central 4₂ se prolonge à son extrémité distale par un troisième manchon tubulaire 4₃ sur la face externe duquel s'ajuste l'extrémité proximale dudit premier tuyau transparent 1. Le compartiment central 4₂ comporte en outre un orifice latéral 5 en forme de quatrième manchon tubulaire sur lequel vient s'adapter un deuxième tuyau souple transparent 6, lui-même assemblé à son autre extrémité à un robinet multi-voies 6.

Des capsules étanches 4, tel que décrit ci-dessus sont commercialisées notamment par la société TERUMO CORPORATION (Japon) sous la marque Radiofocus®.

Ledit second tuyau 6 permet ainsi d'injecter un liquide contenant des substances à visée diagnostique ou thérapeutique à l'intérieur dudit premier tuyau transparent et donc dudit vaisseau, ou encore un liquide de rinçage dudit premier tuyau pour éviter la coagulation du sang qu'il contient, notamment avec du sérum hépariné.

Ledit robinet 6₁ sert à assurer l'obturation dudit second tuyau 6 et donc également dudit premier tuyau 1 auquel il est relié. Il comporte avantageusement plusieurs voies d'introduction pour permettre par exemple le suivi de la mesure de la pression artérielle sur une voie et de l'injection desdits produits à visée diagnostique ou thérapeutique sur une autre voie.

Sur la figure 1, on a représenté une butée d'arrêt 2₃ au niveau de l'aiguille creuse 2₁ qui fait fonction de butée et empêche l'enfoncement excessif éventuel de l'aiguille lors de la ponction.

Sur la figure 2, la partie intermédiaire à profil externe arrondi de l'élément de raccordement constitue un renflement 3₂.

Le dispositif selon l'invention, tel que décrit ci-dessus, a permis d'implanter des endoprothèses Talent® de la société METRONIC AVE (USA) mesurant de 12 à 20 mm de diamètre et de 95 à 110 mm de long une fois déployé dans les vaisseaux.

Les cathéters contenant les endoprothèses étaient de taille de 5,4 mm environ (18 french).

8 porcs fermiers ont été choisis pour l'expérience et traités selon un protocole en accord avec la législation des animaux de laboratoire.

L'animal a été placé en décubitus latéral droit, avec un billot au niveau de la jonction thoraço-abdominale. Les opérateurs se sont positionnés du côté ventral, la colonne vidéo en face côté dorsal.

L'aorte abdominale sous-rénale a été abordée par voie laparoscopique rétropéritonéale après mise en place de 3 trocarts de 10 mm au niveau du flanc gauche, entre la crête iliaque et la 11^{ème} côte. Un rétro pneumo-péritoine a été maintenu à 12 mm Hg durant toute l'intervention. Le segment artériel compris entre l'artère rénale gauche et la trifurcation aortique a été disséqué, sécurisé par 2 lacs avec tirette transpariétale pour permettre un clampage d'amont et d'aval à tout moment pendant le temps endovasculaire. Les artères visibles ont été clipées pour limiter le saignement.

Un guide Terumo® de 0,89 mm(0,035 inch) de diamètre et 180 cm de long a été introduit dans l'espace rétro péritonéal par des trocarts puis sous contrôle laparoscopique dans l'aorte après ponction directe de celle-ci sans clampage. Il a ainsi été positionné dans l'aorte thoracique 60 cm environ en amont.

L'hémostase autour du guide a été maintenu après retrait de celui-ci par une simple pince tenant la paroi aortique. Une dose IV d'héparine a pu être injectée depuis le robinet 6₁.

Un cathéter de 5,4 mm environ (18 french) contenant une endoprothèse a été introduit sur le guide par le même trocart, dans l'espace rétro péritonéal d'abord, puis dans l'aorte sous-rénale par élargissement progressif de l'orifice de ponction sans clampage. La longueur des cathéters a été calculée pour atteindre l'aorte thoracique descendante sans avoir recours à aucun contrôle radiologique et permettre un déploiement de l'endoprothèse entre la sous clavière gauche et le tronc coeliaque. Le largage a été réalisé de manière classique par retrait de la gaine externe du cathéter. Pour ressortir le cathéter de l'aorte abdominale, un clampage en amont et en aval de l'orifice d'introduction par les lacs tirettes a permis de contrôler le saignement. La fermeture aortique a été assurée soit par une bourse réalisée avant la ponction, soit par des points de suture effectuée après le retrait du cathéter.

Après rétablissement de la circulation artérielle et vérification de l'étanchéité de l'aorte, les trocarts de laparoscopie ont été enlevés, les orifices cutanés ont été fermés.

La qualité de la revascularisation en aval de la procédure a été évaluée par le retour à la normale de la courbe oxymétrique enregistrée au niveau de la région caudale de l'animal.

Les porcs ont ensuite été euthanasiés, le positionnement et la perméabilité de l'endoprothèse ont été vérifiés de visu.

L'intervention a été accomplie avec succès chez 7 animaux suivant le protocole établi. Pour les 7 animaux, les pertes sanguines ont été parfaitement maîtrisées. Le saignement moyen a été de 120 ml (de 50 à 210 ml). Les pertes sanguines enregistrées au moment de la ponction ont été négligeables. L'introduction des guides et des différents cathéters n'a pas entraîné de saignement supplémentaire.

Chez 6 animaux, l'abord laparoscopique rétro péritonéal a donné une vue excellente de l'aorte sous rénale. Le temps de dissection aortique a été long, compris entre 92 et 233 minutes.

Dans 2 cas, une ouverture accidentelle du péritoine est survenue. Il a été nécessaire de mettre en place une aiguille à travers la paroi abdominale pour évacuer le pneumo péritoine. Cet incident a rendu l'ensemble de la procédure plus longue, respectivement 240 et 300 minutes.

L'introduction du cathéter de 5,4 mm environ (18 french) dans l'aorte a été difficile 2 fois. Un élargissement de l'orifice d'entrée a été nécessaire avec des ciseaux laparoscopiques après clampage temporaire de l'aorte abdominale.

L'orifice d'introduction aortique est resté étanche, sans artifice supplémentaire pendant que le cathéter de 5,4 mm environ (18 french) était dans l'aorte. Après le retrait de celui-ci, le saignement de la zone d'introduction aortique a été contrôlé par les lacs d'amont et d'aval et la ligature préalable des artères lombaires. Le temps endovasculaire depuis la ponction artérielle, jusqu'au retrait du cathéter, a été rapide. Il a été de 22 minutes en moyenne (de 10 à 35 minutes). Le temps de clampage moyen nécessaire à la fermeture de l'orifice aortique a été de 30 minutes en moyenne, soit de 15 à 70 minutes.

La durée totale de l'intervention a été en moyenne de 205 minutes. Aucun trouble hémodynamique systémique important n'est survenu durant la procédure. Les prélèvements de l'aorte thoracique ont montré dans tous les cas un positionnement et un déploiement correct du dispositif.

L'utilisation d'une voie d'abord laparoscopique des vaisseaux abdominaux paraît intéressante chez les patients ayant des artères fémorales et iliaques en mauvais état. Une pathologie artérielle à ce niveau peut être une contre indication à l'implantation d'une endoprothèse aortique pour anévrisme.

Pour les procédures endovalsculaires thoraciques, une voie laparoscopique permettrait de réduire la distance entre le point d'entrée dans le système artériel et le point de largage. Il en résulterait une diminution des contraintes par frottement ainsi que la possibilité d'utiliser des cathéters de gros calibre. En outre, une diminution du temps d'hospitalisation peut être anticipée en raison de la cicatrisation plus rapide d'un abord laparoscopique par rapport à un abord chirurgical de la fémorale commune.

## Revendications

1. Dispositif chirurgical permettant la ponction d'un vaisseau, notamment d'une artère, et l'introduction endovasculaire d'un guide de radiologie, destiné à être utilisé en chirurgie endovasculaire laparoscopique ou coelioscopique, notamment au niveau des vaisseaux intra-abdominaux, **caractérisé en ce qu'**il comprend un premier tuyau transparent souple (1) coopérant à son extrémité distale avec une aiguille métallique creuse (2) à laquelle il est raccordé, et dans laquelle on peut faire passer un dit guide de radiologie, ledit premier tuyau comprenant à son extrémité proximale un moyen d'obturation (4) dudit premier tuyau, ainsi qu'un moyen d'introduction (4₁) permettant l'introduction étanche dans ledit premier tuyau d'un dit guide de radiologie.

2. Dispositif selon la revendication 1, **caractérisé en ce que** ladite aiguille (2) présente un profil longitudinal courbe.

3. Dispositif selon la revendication 2, **caractérisé en ce que** la courbure de ladite aiguille (2) correspond à une inclinaison de son extrémité distale par rapport à son extrémité proximale solidaire dudit élément de raccordement (2₂), d'un angle de 10 à 45°.

4. Dispositif selon l'une des revendications 1 à 3, **caractérisé en ce que** ladite aiguille métallique creuse (2) comprend :
■ une partie distale à extrémité pointue (2₁)et comprenant un premier canal interne longitudinal creux, de préférence à section transversale circulaire, à travers lequel on peut introduire un dit guide de radiologie, et
■ une partie proximale formant un élément de raccordement (2₂) de ladite aiguille (2) et dudit premier tuyau transparent (1), ledit élément de raccordement (2₂) comportant un deuxième canal interne creux assurant la communication entre l'intérieur dudit premier tuyau transparent (1) et dudit premier canal creux de ladite aiguille, et le diamètre interne dudit deuxième canal interne de l'élément de raccordement (2₂) étant au moins égal au diamètre dudit premier canal creux interne.

5. Dispositif selon la revendication 4, **caractérisé en ce que** ledit premier tuyau transparent (1) présente un diamètre interne supérieur ou égal au diamètre interne dudit deuxième canal interne creux dudit élément de raccordement (2₂), lequel deuxième canal creux comprend une zone de transition à section transversale circulaire en forme d'entonnoir de diamètre diminuant progressivement jusqu'à rejoindre ledit premier canal interne creux de ladite aiguille (2₁).

6. Dispositif selon la revendication 5, **caractérisé en ce que** ledit élément de raccordement (2₂) comprend à son extrémité proximale un premier manchon tubulaire (3₁) sur la surface extérieure duquel vient s'ajuster l'extrémité distale dudit premier tuyau souple transparent (1), ledit premier manchon tubulaire (3₁) se prolongeant à son extrémité distale d'une partie intermédiaire (3₂) assurant la jonction entre ledit premier manchon tubulaire (3₁) et ladite partie distale pointue de l'aiguille (2₁), de telle sorte que ladite partie intermédiaire (3₂) présente une section transversale circulaire de diamètre externe supérieur ou égal à celui du diamètre externe dudit premier manchon tubulaire, et diminuant progressivement depuis sa section transversale de plus grand diamètre jusqu'à sa section transversale au niveau de la jonction avec ladite partyie distale à extrémité pointue de l'aiguille (2₁), ledit deuxième canal interne creux dudit élément de raccordement (2₂) comprenant à l'intérieur de ladite partie intermédiaire (3₂) ladite zone de transition à section transversale diminuant progressivement en forme d'entonnoir.

7. Dispositif selon l'une des revendications 1 à 6, **caractérisé en ce que** ledit premier tuyau transparent (1) comprend à son extrémité proximale une capsule étanche (4) assurant l'obturation dudit premier tuyau souple (1), ladite capsule (4) comportant une membrane souple (4₁) en matériau élastique, incisée apte à être traversée de manière étanche par un dit guide de radiologie.

8. Dispositif selon l'une des revendications 1 à 7, **caractérisé en ce que** ledit premier tuyau souple (1) comporte un moyen d'injection (5) permettant d'injecter un liquide dans ledit premier tuyau souple (1), constitué de préférence d'un orifice latéral (5) dans la zone proximale dudit premier tuyau destinée à rester à l'extérieur dudit patient, ledit orifice latéral (5) étant de préférence intégré, le cas échéant, à une dite capsule étanche (4).

9. Dispositif selon la revendication 8, **caractérisé en ce que** ledit moyen d'injection (5) comprend un second tuyau souple (6) adaptable sur ledit orifice latéral (5) et comportant à son extrémité libre un robinet (6₁) de préférence à plusieurs voies.

10. Ensemble de différents éléments permettant d'obtenir après assemblage desdits éléments, un dispositif selon l'une des revendications précédentes, **caractérisé en ce que** lesdits éléments comprennent :
- ledit premier tuyau transparent souple (1),
- ladite aiguille métallique creuse (2),
- le cas échéant, ledit moyen d'obturation, de préférence ladite capsule étanche (4),
- le cas échéant, ledit moyen d'injection, de préférence ledit second tuyau souple (6), comportant de préférence encore, ledit robinet (6₁), lesdits éléments étant préassemblés ou désassemblés, au moins pour partie, de préférence dans un même conditionnement.

## Claims

1. A surgical device for pricking a vessel, notably an artery, and for endovascular introduction of a radiology guide, intended to be used in laparoscopic or coelioscopic endovascular surgery, notably on intra-abdominal vessels, **characterized in that** it comprises a first flexible transparent tube (1) cooperating at its distal end with a hollow metal needle (2) to which it is connected, and in which a so-called radiology guide may be inserted, said first tube comprising at its proximal end a means (4) for sealing said first tube, as well as an introduction means for sealably introducing a so-called radiology guide into said first tube.

2. The device according to claim 1, **characterized in that** said needle (2) has a curved longitudinal profile.

3. The device according to claim 2, **characterized in that** the curvature of said needle (2) corresponds to an inclination of its distal end relative to its proximal end integral with said connecting element (2₂), in an angle from 10 to 45°.

4. The device according to any of claims 1 to 3, **characterized in that** said hollow metal needle (2) comprises:
- a distal portion with a pointed end (2₁) and comprising a first hollow longitudinal internal channel, preferably with a circular cross-section, through which a so-called radiology guide may be introduced, and
- a proximal portion forming a connecting element (2₂) of said needle (2) and of said first transparent tube (1), said connecting element (2₂) comprising a second hollow internal channel providing communication between the inside of said first transparent tube (1) and of said first hollow channel of said needle, and the internal diameter of said second internal channel of the connecting element (2₂) being at least equal to the diameter of said first internal hollow channel.

5. The device according to claim 4, **characterized in that** said first transparent tube (1) has an internal diameter larger than or equal to the internal diameter of said second hollow internal channel of said connecting element (2₂), said second hollow channel comprises a funnel-shaped transition area with a circular cross-section of a diameter which gradually decreases until it joins said first hollow internal channel of said needle (2₁) .

6. The device according to claim 5, **characterized in that** said connecting element (2₂) comprises at its proximal end a first tubular sleeve (3₁) on the outer surface of which the distal end of said first transparent flexible tube (1) will be fitted, said first tubular sleeve (3₁) extending at its distal end with an intermediate portion (3₂) providing the junction between said first tubular sleeve (3₁) and said pointed distal portion of the needle (2₁), so that said intermediate portion (3₂) has a circular cross-section with an outer diameter larger than or equal to that of the outer diameter of said first tubular sleeve, and gradually decreasing from its cross-section of larger diameter right up to its cross-section at the junction with said distal portion of the needle (2₁), with a pointed end, said second hollow internal channel of said connecting element (2₂) comprising inside said intermediate portion (3₂) said funnel-shaped transition area with a gradually decreasing cross-section.

7. The device according to any of claims 1 to 6, **characterized in that** said first transparent tube (1) comprises at its proximal end a sealed capsule (4) providing the seal of said first flexible tube (1), said capsule (4) including a flexible membrane (4₁) in an elastic material, incised so as to be able to be sealably crossed by a so-called radiology guide.

8. The device according to any of claims 1 to 7, **characterized in that** said first flexible tube (1) includes an injection means (5) for injecting a liquid into said first flexible tube (1), preferably consisting of a side orifice (5) in the proximal area of said first tube intended to remain on the outside of said patient, said side orifice (5) being preferably integrated to a said sealed capsule (4) if necessary.

9. The device according to claim 8, **characterized in that** said injection means (5) comprises a second flexible tube (6) which may be adapted on said side orifice (5) and including at its free end a valve (6₁), preferably a multi-way valve.

10. A set of different elements with which after assembling said elements, a device according to any of the preceding claims may be obtained, **characterized in that** said elements comprise:
- said first flexible transparent tube (1),
- said hollow metal needle (2),
- if necessary, said sealing means, preferably said sealed capsule (4),
- if necessary, said injection means, preferably said second flexible tube (6), comprising still more preferably said valve (6₁),
said elements being preassembled or disassembled, at least in part, preferably in a same package.

## Patentansprüche

1. Chirurgische Vorrichtung, die die Punktion eines Gefäßes, insbesondere einer Arterie, und die endovaskuläre Einführung eines Röntgenleiters ermöglicht, und die dazu bestimmt ist, in der laparoskopischen oder zölioskopischen endovaskulären Chirurgie, insbesondere auf Höhe der intraabdominalen Gefäße, verwendet zu werden, **dadurch gekennzeichnet, dass** sie einen ersten biegsamen, transparenten Schlauch
(1) umfasst, der an seinem distalen Ende mit einer hohlen Metallnadel (2) zusammenwirkt, mit welcher er verbunden ist und in welcher man einen besagten Röntgenleiter passieren lassen kann, wobei der besagte erste Schlauch an seinem proximalen Ende ein Mittel zum Verschluss (4) des besagten ersten Schlauches umfasst, sowie ein Einführmittel (4₁), das die dichte Einführung eines besagten Röntgenleiters in den besagten ersten Schlauch ermöglicht.

2. Vorrichtung nach Anspruch 1, **dadurch gekennzeichnet, dass** die besagte Nadel (2) ein gekrümmtes Längsprofil aufweist.

3. Vorrichtung nach Anspruch 2, **dadurch gekennzeichnet, dass** die Krümmung der besagten Nadel (2) einer Neigung ihres distalen Endes in Bezug auf ihr proximales Ende, das mit dem besagten Anschlusselement (2₂) verbunden ist, um einen Winkel von 10 bis 45° entspricht.

4. Vorrichtung nach einem der Ansprüche 1 bis 3, **dadurch gekennzeichnet, dass** die besagte hohle Metallnadel (2) Folgendes umfasst:
- einen distalen Teil mit spitzem Ende (2₁), der einen ersten hohlen, inneren Längskanal umfasst, vorzugsweise mit kreisförmigem Querschnitt, durch den man einen besagten Röntgenleiter einführen kann, und
- einen proximalen Teil, der ein Anschlusselement (2₂) von der besagten Nadel (2) und dem besagten ersten transparenten Schlauch (1) bildet, wobei das besagte Anschlusselement (2₂) einen zweiten hohlen Innenkanal umfasst, der die Verbindung zwischen dem Inneren des besagten ersten transparenten Schlauches (1) und des besagten ersten hohlen Kanals der besagten Nadel sicherstellt, und wobei der Innendurchmesser des besagten zweiten Innenkanals des Anschlusselements (2₂) mindestens so groß ist wie der Durchmesser des besagten ersten hohlen Innenkanals.

5. Vorrichtung nach Anspruch 4, **dadurch gekennzeichnet, dass** der besagte erste transparente Schlauch (1) einen Innendurchmesser aufweist, der größer oder mindestens so groß ist wie der Innendurchmesser des besagten zweiten hohlen Innenkanals des besagten Anschlusselements (2₂), wobei dieser zweite hohle Kanal einen Übergangsbereich mit kreisförmigem Querschnitt und Trichterform umfasst, deren Durchmesser allmählich abnimmt, bis er mit dem besagten ersten hohlen Innenkanal der besagten Nadel (2₁) zusammentrifft.

6. Vorrichtung nach Anspruch 5, **dadurch gekennzeichnet, dass** das besagte Anschlusselement (2₂) an seinem proximalen Ende eine erste röhrenförmige Hülse (3₁) umfasst, an deren äußere Oberfläche sich das distale Ende des besagten ersten biegsamen, transparenten Schlauches (1) anlegt, wobei sich die besagte erste röhrenförmige Hülse (3₁) an ihrem distalen Ende um einen Zwischenteil (3₂) verlängert, der die Verbindung zwischen der besagten ersten röhrenförmigen Hülse (3₁) und dem besagten spitzen Distalteil der Nadel (2₁) sicherstellt, und zwar so, dass der besagte Zwischenteil (3₂) einen kreisförmigen Querschnitt mit einem Außendurchmesser aufweist, der größer oder mindestens so groß ist wie der Außendurchmesser der besagten ersten röhrenförmigen Hülse, und wobei er allmählich abnimmt ausgehend von seinem Querschnitt mit dem größten Durchmesser bis zu seinem Querschnitt auf Höhe der Verbindung mit dem besagten spitzen Distalteil der Nadel (2₁), wobei der besagte zweite hohle Innenkanal des besagten Anschlusselements (2₂) im Inneren des besagten Zwischenteils (3₂) den besagten Übergangsbereich umfasst, dessen Querschnitt in der Form eines Trichters allmählich abnimmt.

7. Vorrichtung nach einem der Ansprüche 1 bis 6, **dadurch gekennzeichnet, dass** der besagte erste transparente Schlauch (1) an seinem proximalen Ende eine dichte Kapsel (4) umfasst, die den Verschluss des besagten ersten biegsamen Schlauches (1) sicherstellt, wobei die besagte Kapsel (4) eine biegsame Membran (4₁) aus elastischem Material umfasst, die eingeschnitten und imstande ist, in abgedichteter Weise von einem besagten Röntgenleiter durchdrungen zu werden.

8. Vorrichtung nach einem der Ansprüche 1 bis 7, **dadurch gekennzeichnet, dass** der besagte erste biegsame Schlauch (1) ein Injektionsmittel (5) umfasst, das es ermöglicht, eine Flüssigkeit in den besagten ersten biegsamen Schlauch (1) zu injizieren, und das vorzugsweise aus einer seitlichen Öffnung (5) im proximalen Bereich des besagten ersten Schlauches besteht, der dazu bestimmt ist, außerhalb des besagten Patienten zu verbleiben, wobei die besagte seitliche Öffnung (5) vorzugsweise und gegebenenfalls in eine besagte dichte Kapsel (4) integriert wird.

9. Vorrichtung nach Anspruch 8, **dadurch gekennzeichnet, dass** das besagte Injektionsmittel (5) einen zweiten biegsamen Schlauch (6) umfasst, der auf der besagten seitlichen Öffnung (5) angepasst werden kann und an seinem freien Ende einen Hahn (6₁), vorzugsweise einen Mehrwegehahn, umfasst.

10. Einheit aus verschiedenen Elementen, die es ermöglicht, nach Zusamnensetzen der besagten Elemente eine Vorrichtung nach einem der vorhergehenden Ansprüche zu erhalten, **dadurch gekennzeichnet, dass** die besagten Elemente Folgendes umfassen:
- den besagten ersten biegsamen, transparenten Schlauch (1),
- die besagte hohle Metallnadel (2),
- gegebenenfalls das besagte Verschlussmittel, vorzugsweise die besagte dichte Kapsel (4),
- gegebenenfalls das besagte Injektionsmittel, vorzugsweise den besagten zweiten biegsamen Schlauch (6), der vorzugsweise außerdem den besagten Hahn (6₁) umfasst,
wobei die besagten Elemente vorzugsweise in ein und derselben Verpackung zumindest teilweise vormontiert oder demontiert sind.
